# EUROPÄISCHE PATENTANMELDUNG

(11) **EP 1 281 705 A2**
(43) Veröffentlichungstag der Anmeldung: **05.02.2003**
(21) Anmeldenummer: 02016267.3
(22) Anmeldetag: 23.07.2002
(51) Int. Cl.: C07D 301/08, C07D 301/10

(54) **Verfahren zur Herstellung von Epoxiden aus Alkenen**

(30) Priorität: 02.08.2001 DE 10137783
(71) Anmelder: BAYER AG, 51368 Leverkusen (DE)
(72) Erfinder: Seeba, Johann, Dr., 51379 Leverkusen (DE); Nagy, Anton-Joseph, Dr., 1016VX Amsterdam (NL); Völkening, Stephan, Dr., 50670 Köln (DE); Wiessmeier, Georg, Dr., 51467 Bergisch Gladbach (DE)

(57) **Zusammenfassung**

Die Erfindung betrifft ein Verfahren zur Herstellung von Epoxiden durch Oxidation von Kohlenwasserstoffen in Gegenwart von Sauerstoff und wenigstens einem Reduktionsmittel und einem Katalysator, dadurch gekennzeichnet, dass man die Reaktionsmischung durch mindestens eine Katalysator enthaltende Schicht und durch mindestens eine Adsorptionsmittel enthaltende Schicht, in der das Epoxid adsorbiert wird, leitet und die Katalysator enthaltenden Schichten und die Adsorptionsmittel enthaltenden Schichten abwechselnd hintereinander angeordnet sind.

## Beschreibung

Die Erfindung betrifft ein Verfahren zur Herstellung von Epoxiden durch Direktoxidation von Kohlenwasserstoffen mit Sauerstoff in der Gasphase in Gegenwart wenigstens eines Reduktionsmittels und eines Katalysators. Wesentliches Merkmal der Erfindung ist, dass man die Reaktionsmischung durch mindestens eine Katalysator enthaltende Schicht und durch mindestens eine Adsorptionsmittel enthaltende Schicht, in der das Epoxid adsorbiert wird, leitet und die Katalysator enthaltenden Schichten und die Adsorptionsmittel enthaltenden Schichten abwechselnd hintereinander angeordnet sind.

Die Direktoxidation von Ethen zu Ethenoxid durch molekularen Sauerstoff ist gut bekannt und wird kommerziell zur Produktion von Ethenoxid in der Gasphase angewendet. Der typische Katalysator für diese Anwendung enthält metallisches oder ionisches Silber, eventuell noch modifiziert mit verschiedenen Promotoren und Aktivatoren. Die meisten dieser Katalysatoren enthalten einen porösen, inerten Katalysatorträger mit geringen Oberflächen wie z.B. alpha-Aluminiumoxid, auf den Silber und Promotoren aufgebracht wurden. Ein Überblick zur Direktoxidation von Ethen in Gegenwart von geträgerten Silberkatalysatoren wurde von Sachtler et al. in Catalysis Reviews: Science and Engineering, 23 (1&2), 127-149 (1981) zusammengestellt.

In US-B-5 623 090 wird eine Gasphasen-Direktoxidation von Propen zu Propenoxid mit relativ kleinen Propenumsätzen (0,5-1 % Propenumsatz bezogen auf eine 10 %ige Propen-Feedkonzentration), aber Propenoxid-Selektivitäten von > 90 % mit Sauerstoff als Oxidationsmittel beschrieben. Es handelt sich hierbei um eine Gold-Titandioxid-katalysierte Gasphasenoxidation mit molekularem Sauerstoff in Gegenwart von Wasserstoff bei Temperaturen von 40-70°C. Als Katalysator wird handelsübliches kristallines Titandioxid mit vorwiegend Anatasmodifikation (P 25, Degussa; 70 % Anatas u. 30 % Rutil) verwendet.

Bekannt sind weiterhin Katalysatoren, bei denen Goldpartikel auf einen Träger, bestehend aus dispergierten Titanoxidzentren auf einer rein anorganischen Siliziummatrix aufgebracht werden (WO-98/00415-A1; WO-98/00414-A1, WO-99/43431-A1, EP-A1-0 827 779).

Die genannten Verfahren haben neben den relativ geringen Propenumsätzen den großen Nachteil, dass die offenbarten Katalysatoren mit der Zeit stark desaktivieren. Typische Halbwertszeiten bei Normaldruck und 50°C liegen bei 30-150 Minuten. Temperatur- und/oder Druckerhöhung zur Steigerung des Umsatzes verkürzen die Halbwertszeiten weiter. Dies ist auf eine Folgereaktion der entstandenen Produkte (Propenoxid und Wasser) mit dem Katalysator zurückzuführen (Bildung von Glykolen). Dadurch werden die für die Reaktion essentiellen sogenannten aktiven Zentren des Katalysators mit Folgeprodukten belegt und stehen nicht für weitere Umsetzungen von Propen zu Propenoxid zur Verfügung.

Es ist also eine Entwicklung eines Verfahrens wünschenswert, bei dem die Desaktivierung der Katalysatoren verhindert oder zumindest stark unterdrückt wird.

Verfahren zur Oxidation von Alkenen mit Sauerstoff bzw. Luft unter Verwendung geeigneter Katalysatoren und Adsorbenzien zur Gewinnung entsprechender Epoxide sind ebenfalls bereits bekannt (EP-A1-0 372 972, EP-A1-0 328 280, EP-A1-0 336 592, US-A-4 990 632, EP-A1-0 467 538, EP-A1-0 646 558). In den zitierten Dokumenten wird jedoch nicht das Epoxid durch Adsorption entfernt sondern der Alkenstrom von Verunreinigungen wie Reaktionsprodukten (z.B. CO, CO2 oder niederen Kohlenwasserstoffen) gereinigt. Der so gereinigte Alkenstrom kann dann recycliert und für die Reaktion wieder eingesetzt werden.

US-A 5 117 012 beschreibt die Adsorption von Epoxybutadien bei der Oxidation von Butadien.

Die Aufgabe der vorliegenden Erfindung bestand darin, ein verbessertes Verfahren zur Herstellung von Epoxiden aus Alkenen in Gegenwart von Sauerstoff und einem Reduktionsmittel zur Verfügung zu stellen, bei dem die Katalysatordesaktivierung verringert und die Ausbeute erhöht wird.

Es wurde nun ein Verfahren zur Herstellung von Epoxiden durch Oxidation von Alkenen in Gegenwart von Sauerstoff und einem Reduktionsmittel und eines Katalysators gefunden, das durch die Adsorption von Produkt niedrige Produktkonzentrationen im Gasraum und am Katalysator gewährleistet und dadurch die Desaktivierung des Katalysators reduziert und die Ausbeute an Epoxid steigert.

Die gestellte Aufgabe wird gelöst durch ein Verfahren zur Herstellung von Epoxiden durch Oxidation von Kohlenwasserstoffen in Gegenwart von Sauerstoff und wenigstens einem Reduktionsmittel und einem Katalysator, dadurch gekennzeichnet, dass man die Reaktionsmischung durch mindestens eine Katalysator enthaltende Schicht und anschließend durch mindestens eine Adsorptionsmittel enthaltende Schicht, in der das Epoxid adsorbiert wird, leitet und, für den Fall, dass mehr als eine Katalysator enthaltende Schicht oder mehr als eine Adsorptionsmittel enthaltende Schicht vorhanden ist, die Katalysator enthaltenden Schichten und die Adsorptionsmittel enthaltenden Schichten abwechselnd hintereinander angeordnet sind.

Hierbei bedeutet "hinter" oder "hinter einander" immer in Strömungsrichtung weiter hinten.

Wenn nur eine Katalysator enthaltende Schicht vorhanden ist, dann wird das Reaktionsgas bevorzugt nach dem Durchströmen der Schichten zurückgeführt, um sie erneut zu durchströmen.

Unter dem Begriff Kohlenwasserstoff werden ungesättigte oder gesättigte Kohlenwasserstoffe wie Olefine oder Alkane verstanden, die auch Heteroatome wie N, O, P, S oder Halogene enthalten können. Die zu oxidierende organische Komponente kann azyklisch, monozyklisch, bizyklisch oder polyzyklisch und kann monoolefinisch, diolefinisch oder polyolefinisch sein.

Bei Kohlenwasserstoffen mit zwei oder mehreren Doppelbindungen können die Doppelbindungen konjugiert und nichtkonjugiert vorliegen. Bevorzugt werden Kohlenwasserstoffe oxidiert, aus denen solche Oxidationsprodukte gebildet werden, deren Partialdruck niedrig genug liegt, um das Produkt ständig vom Katalysator zu entfernen. Bevorzugt sind ungesättigte und gesättigte Kohlenwasserstoffe mit 2 bis 20, vorzugsweise 2 bis 12 Kohlenwasserstoffatomen, insbesondere Ethen, Ethan, Propen, Propan, Isobutan, Isobutylen, 1-Buten, 2-Buten, cis-2-Buten, trans-2-Buten, 1,3-Butadien, Pentene, Pentan, 1-Hexen, Hexene, Hexan, Hexadien, Cyclohexen, Benzol.

Das Verfahren kann eine große Zahl von Katalysator enthaltenden Schichten, vorzugsweise 2 bis 20, besonders bevorzugt 3 bis 10 Schichten und eine große Zahl von Adsorptionsmittel enthaltenden Schichten, vorzugsweise 2 bis 20, besonders bevorzugt 3 bis 10 Schichten enthalten.

Dabei können die Katalysator und die Adsorptionsmittel enthaltenden Schichten in einem oder mehreren, z.B. seriell verschalteten Reaktoren, angeordnet sein.

In einer bevorzugten Ausführungsform des Verfahrens sind hinter jeder Katalysator enthaltenden Schicht mehrere, bevorzugt 2 bis 10, besonders bevorzugt 2 bis 5 parallel angeordnete Adsorptionsmittel enthaltende Schichten angeordnet, die abwechselnd für Adsorption und Desorption des Reaktionsprodukts genutzt werden können. In einer besonderen Ausführungsform gibt es eine letzte Katalysator enthaltende Schicht, hinter der keine Adsorptionsmittel enthaltende Schichten angeordnet sind.

Ein Merkmal des vorliegenden Verfahrens besteht darin, dass in jeder Katalysator enthaltenden Schicht das Alken nur teilweise umgesetzt wird. Vorzugsweise wird in jeder Katalysatorschicht nur 0,01 bis 90 %, vorzugsweise 1 bis 50 %, besonders bevorzugt 2 bis 30 % des maximal möglichen Umsatzes umgesetzt. Dadurch wird erreicht, dass das Reaktionsprodukt in der nachfolgend durchströmten Adsorptionsmittel enthaltenden Schicht aus dem Reaktionsgemisch ausgeschleust wird und dadurch die Selektivitätsverbesserung und Standzeitverlängerung des Katalysators stattfinden können. Der maximal mögliche Umsatz kann z.B. durch das thermodynamische Gleichgewicht vorgegeben sein.

Der Sauerstoff kann in verschiedenster Form eingesetzt werden, z.B. molekularer Sauerstoff, Luft und Stickstoffoxid. Molekularer Sauerstoff wird bevorzugt.

Reduktionsmittel im Sinne der Erfindung ist eine Verbindung, die ein Sauerstoffatom aufnehmen oder ein Wasserstoffatom abgeben kann wie Wasserstoff, Kohlenmonoxid oder Synthesegas. Bevorzugt ist Wasserstoff und Kohlenmonoxid.

Es kann jede bekannte Wasserstpffquelle genutzt werden, wie z.B. reiner Wasserstoff, Cracker-Wasserstoff, Synthesegas oder Wasserstoff aus Dehydrierung von Kohlenwasserstoffen und Alkoholen. In einer anderen Ausführungsform der Erfindung kann der Wasserstoff auch in einem vorgeschalteten Reaktor in situ erzeugt werden, z.B. durch Dehydrierung von Propan oder Isobutan oder Alkoholen wie Isobutanol. Der Wasserstoff kann auch als Komplex-gebundene Spezies, z.B. Katalysator-Wasserstoffkomplex, in das Reaktionssystem eingeführt werden.

Zu den essentiell notwendigen oben beschriebenen Eduktgasen kann optional auch ein Verdünnungsgas, wie Stickstoff, Helium, Argon, Methan, Kohlendioxid, oder ähnliche, sich überwiegend inert verhaltende Gase, eingesetzt werden. Auch Mischungen der beschriebenen Inertkomponenten können eingesetzt werden. Der Inertkomponentenzusatz ist zum Transport der freiwerdenden Wärme dieser exothermen Oxidationsreaktion und aus sicherheitstechnischen Gesichtspunkten oft günstig. Wird der erfindungsgemäße Prozess in der Gasphase durchgeführt, werden bevorzugt gasförmige Verdünnungskomponenten wie z.B. Stickstoff, Helium, Argon, Methan und evtl. Wasserdampf und Kohlendioxid verwendet. Wasserdampf und Kohlendioxid sind zwar nicht völlig inert, bewirken aber bei kleinen Konzentrationen (< 2 Vol.-% des gesamten Gasstromes) häufig einen positiven Effekt.

Das relative molare Verhältnis von Kohlenwasserstoff, Sauerstoff, Reduktionsmittel (insbesondere Wasserstoff) und optional einem Verdünnungsgas ist in weiten Bereichen variierbar.

Bevorzugt wird im Bereich maximal 30 Mol-%, bevorzugt von 1-30 Mol-%, besonders bevorzugt 5-25 Mol-% Sauerstoff eingesetzt (Mol-%, bezogen auf den gesamten Gasstrom).

Bevorzugt wird ein Überschuss von Kohlenwasserstoff, bezogen auf eingesetzten Sauerstoff (auf molarer Basis) eingesetzt. Der Kohlenwasserstoffgehalt liegt typischerweise größer 1 Mol-% und kleiner als 96 Mol-% (bevorzugt auf den gesamten Gasstrom). Bevorzugt werden Kohlenwasserstoffgehalte im Bereich von 5 bis 90 Mol-%, besonders bevorzugt von 20 bis 85 Mol-% eingesetzt. Der molare Reduktionsmittelanteil (insbesondere Wasserstoffanteil) - in Bezug auf die Gesamtmolzahl aus Kohlenwasserstoff, Sauerstoff, Reduktionsmittel und Verdünnungsgas - kann in weiten Bereichen variiert werden. Typische Reduktionsmittelgehalte liegen bei größer als 0,1 Mol-%, bevorzugt bei 2-80 Mol-%, besonders bevorzugt bei 3-70 Mol-%.

Der in dem Verfahren in der Katalysator enthaltenden Schicht eingesetzte Katalysator ist bevorzugt ein Metall-Katalysator auf einem oxidischen Träger. Als Metalle sind die Elemente Cobalt, Ruthenium, Iridium, Nickel, Palladium, Platin, Kupfer, Silber und Gold, bevorzugt Gold, geeignet. Auch Kombinationen dieser Edelmetalle sind möglich. Als Trägermaterialien sind die Oxide von metallischen und halbmetallischen Elementen geeignet. Die Träger können auch aus Oxiden verschiedener metallischer oder halbmetallischer Elemente bestehen. Bevorzugte Trägermaterialien sind z.B.

Titanoxide und Gemische von Titanoxiden und Siliziumoxiden. Solche Katalysatoren sind beispielsweise in DE-A1-199 59 525, DE-A1-100 23 717 offenbart, welche hiermit für die Zwecke der US-Patentpraxis als Referenz in die vorliegende Anmeldung aufgenommen werden.

Dabei kann die Katalysator enthaltende Schicht auch weitere Katalysatoren in geringem Umfang oder inerte Bestandteile zur Katalysatorverdünnung enthalten.

Vorteilhaft wird die Oxidationsreaktion bei erhöhten Reaktionsdrücken durchgeführt. Bevorzugt sind Reaktionsdrücke von > 1 bar, besonders bevorzugt 2-50 bar.

Die Katalysatorbelastung kann in weiten Bereichen variiert werden. Bevorzugt werden Katalysatorbelastungen im Bereich von 0,5-100 1 Gas (gesamter Gasstrom, d.h. Reaktionsmischung) pro ml Katalysator und Stunde verwendet, besonders bevorzugt werden Katalysatorbelastungen von 2-50 1 Gas pro ml Katalysator und Stunde gewählt.

Bei der katalytischen Oxidation von Kohlenwasserstoffen in Gegenwart von Wasserstoff entsteht in der Regel Wasser als Koppelprodukt zum entsprechenden selektiven Oxidationsprodukt.

Die kontinuierliche Trennung der bei der Direkt-Oxidation in Gegenwart von Sauerstoff und einem Reduktionsmittel entstehenden Partialoxidationsprodukte vom Reaktionsgemisch gelingt überraschend auch in Gegenwart von Wasser und/oder Wasserdampf und sauer reagierenden Nebenprodukten durch selektive Adsorption an geeigneten Adsorptionsmitteln ohne Zersetzung dieser Adsorptionsprodukte.

Als Adsorptionsmittel kommen daher alle Feststoffe in Frage, die in der Lage sind, partiell oxidierte Kohlenwasserstoffe ohne Zersetzung auch in Gegenwart von Wasser und/oder Wasserdampf und sauer reagierenden Nebenprodukten zu adsorbieren. Die Adsorptionsmittel enthaltende Schicht darf dabei keine Folgereaktionen der adsorbierten Partialoxidationsprodukte initiieren.

Als Adsorptionsmittel sind beispielsweise Zeolithe geeignet. Bevorzugt werden hydrophobe Zeolithe eingesetzt. Besonders bevorzugt sind Zeolithe des Typs Faujasit (HY) mit einem niedrigen Aluminiumanteil (z.B. Wessalith DAY oder DAZ F20 der Fa. Degussa). Es können aber auch Molekularsiebe oder andere Substanzen verwendet werden, auf denen bevorzugt das Epoxid adsorbiert und von dem es unzersetzt durch Desorption oder Waschung wieder entfernt werden kann.

Das erfindungsgemäße Verfahren dient insbesondere der Herstellung von Epoxiden aus den entsprechenden Alkenen. Bevorzugte Alkene sind Ethen, Propen und Buten (z.B. 1-Buten, 2-Buten), besonders bevorzugt Propen.

Das Verfahren kann je nach Wahl des eingesetzten Katalysators bei Temperaturen im Bereich von 20 bis 400°C, vorzugsweise 20 bis 200°C ausgeführt werden. Bei Temperaturen größer 220°C werden neben Partialoxidationsprodukten größere Mengen Kohlendioxid gebildet.

Ein Vorteil des erfindungsgemäßen Verfahrens ist die Steigerung der Ausbeute durch den Einsatz von Adsorptionsmittel enthaltenden Schichten. Das Adsorptionsmittel erniedrigt die Epoxid-Konzentration im Gasraum und am Katalysator und reduziert dadurch die Desaktivierung des Katalysators und steigert somit die Ausbeute an Epoxid.

### Beispiele

### Beispiel 1: Herstellung des genannten Katalysators: Au / TiO₂:

Zur Suspension von 10 g Titanoxidhydrat (BET-Oberfläche von 380 m²/g, 12 % Wasser) in 0,3 l deionisiertem Wasser werden bei Raumtemperatur unter Rühren 100 mg H(AuCl₄) x H₂O, gelöst in 100 ml deionisiertem Wasser, innerhalb von 60 min zugetropft. Zur Fällung des Goldhydroxides wird mit einer 0,5 molaren Na₂CO₃-Lösung der pH-Wert auf 8 eingestellt; die schwach gelbe Suspension entfärbt sich. Die Suspension wird 3 h bei Raumtemperatur gerührt, der Feststoff abgetrennt und 4 mal mit je 25 ml vollentsalztem gewaschen. Zur Trocknung wird der Feststoff 2 h bei 150°C und 1 h bei 200°C gehalten, und anschließend wird der getrocknete Kontakt an der Luft 2 h bei 250°C und 5 h bei 400°C kalziniert.

Man erhält einen Katalysator mit 0,5 Gew.-% Gold. Die Charakterisierung mit TEM (Transmissions-Elektronenmikroskopie) ergibt nanoskalige Goldpartikel mit mittleren Teilchendurchmessern von ca. 1-6 nm.

### Beispiel 2: Vergleich des erfindungsgemäßen Verfahrens mit dem Stand der Technik unter Verwendung des Katalysators aus Beispiel 1

Es wurden zwei Metallrohrreaktoren mit 10 mm Innendurchmesser und 20 cm Länge eingesetzt, welche mittels eines Ölthermostaten temperiert wurden. Die Reaktoren wurden mit einem Satz von vier Massendurchflussreglern (Kohlenwasserstoff, Sauerstoff, Wasserstoff, Stickstoff) mit Eduktgasen versorgt. Zur Reaktion wurden zwei Reaktoren mit jeweils 500 mg eines Katalysators bestehend aus 0,5 % metallischem Gold in Form kleiner Partikel (2-10 nm) auf einem Titandioxid (AI 5585) geträgert und 2g Zeolith (Wessalith DAZ F20 der Fa. Degussa) befüllt. Im ersten Reaktor wurde der Katalysator in einer Schicht eingebracht, gefolgt von einer Schicht Zeolith (Vergleich). Im zweiten Reaktor wurde die Katalysator- und Zeolithmenge in 4 gleich große Teile aufgeteilt und in Form alternierender Schichten Katalysator/Zeolith eingebracht (erfindungsgemäß). Die Reaktoren wurden auf eine Temperatur von 50°C gebracht.

Die Eduktgase wurden von oben in den Reaktor eindosiert. Die Standardkatalysatorbelastung lag bei 3 l Gas / (g Kat. * h). Als "Standardkohlenwasserstoff" wurde Propen ausgewählt. Zur Durchführung der Oxidationsreaktionen wurde ein mit Stickstoff angereicherter Gasstrom folgender Zusammensetzung ausgewählt: N₂ / H₂ / O₂ / C₃H₆ = 14 / 75 / 5 / 6. Die Angaben sind in Vol.-% unter Standardbedingungen. Die Reaktionsgase wurden gaschromatographisch quantitativ analysiert. Die gaschromatographische Auftrennung der einzelnen Reaktionsprodukte erfolgte durch eine kombinierte FID/WLD-Methode, bei der drei Kapillarsäulen durchlaufen werden:
- FID:: HP-Innowax®, 0,32 mm Innendurchmesser, 60 m lang, 0,25 um Schichtdicke (FID heißt Flammen-Ionisations-Detektor).
- WLD:: Hintereinanderschaltung von HP-Plot® Q, 0,32 mm Innendurchmesser, 30 m lang, 20 µm Schichtdicke HP-Plot® Molsieve 5 A, 0,32 mm Innendurchmesser, 30 m lang, 12 µm Schichtdicke (WLD heißt Wärmeleitfähigkeit-Detektor).

Die Reaktion wurde über einen Zeitraum von zwei Stunden betrieben. Anschließend wurde im System mit den alternierenden Schichten auf den Zeolithschichten bei der thermogravimetrischen Analyse um 60 % mehr Propenoxid gefunden als im Zweischichten-System.

Am Ausgang des Reaktors trat wie erwartet kein Propenoxid mehr auf. Das adsorbierte Propenoxid wurde bei Erhöhung der Temperatur wieder freigesetzt.

## Patentansprüche

1. Verfahren zur Herstellung von Epoxiden durch Oxidation von Kohlenwasserstoffen in Gegenwart von Sauerstoff und wenigstens einem Reduktionsmittel und einem Katalysator, **dadurch gekennzeichnet, dass** man die Reaktionsmischung durch mindestens eine Katalysator enthaltende Schicht und anschließend durch mindestens eine Adsorptionsmittel enthaltende Schicht, in der das Epoxid adsorbiert wird, leitet und für den Fall, dass mehr als eine Katalysator enthaltende Schicht oder mehr als eine Adsorptionsmittel enthaltende Schicht vorhanden ist, die Katalysator enthaltenden Schichten und die Adsorptionsmittel enthaltenden Schichten abwechselnd hintereinander angeordnet sind.

2. Verfahren zur Herstellung von Epoxiden durch Oxidation von Kohlenwasserstoffen in Gegenwart von Sauerstoff und wenigstens einem Reduktionsmittel und einem Katalysator nach Anspruch 1, **dadurch gekennzeichnet, dass** man die Reaktionsmischung durch mindestens zwei Katalysator enthaltende Schichten und durch mindestens eine Adsorptionsmittel enthaltende Schicht, in der das Epoxid adsorbiert wird, leitet und die Katalysator enthaltenden Schichten und die Adsorptionsmittel enthaltenden Schichten abwechselnd hintereinander angeordnet sind.

3. Verfahren nach Anspruch 1 oder 2, **dadurch gekennzeichnet, dass** hinter jeder Katalysator enthaltenden Schicht, mehrere, bevorzugt 2 bis 10, besonders bevorzugt 2 bis 5 parallel angeordnete Adsorptionsmittel enthaltende Schichten angeordnet sind, die abwechselnd für Adsorption und Desorption des Reaktionsprodukts genutzt werden können.

4. Verfahren nach Anspruch 1 bis 3, **dadurch gekennzeichnet, dass** in jeder Katalysator enthaltenden Schicht der Kohlenwasserstoff nur teilweise umgesetzt wird.

5. Verfahren nach Anspruch 1 bis 4, **dadurch gekennzeichnet, dass** die Katalysator enthaltende Schicht einen Metall - Katalysator auf Metalloxid-Träger enthält, wobei das Metall ausgewählt wird aus der Gruppe bestehend aus Cobalt, Ruthenium, Iridium, Nickel, Palladium, Platin, Kupfer, Silber und Gold oder Mischungen aus 2 oder mehreren Metallen daraus.

6. Verfahren nach Anspruch 1 bis 5, **dadurch gekennzeichnet, dass** als Adsorptionsmittel hydrophobe Zeolithe eingesetzt werden.

7. Verfahren nach Anspruch 1 bis 6, **dadurch gekennzeichnet, dass** das Alken aus der Gruppe bestehend aus Ethen, Propen, Buten ausgewählt wird.

8. Verfahren nach Anspruch 1 bis 7, **dadurch gekennzeichnet, dass** als Reduktionsmittel Wasserstoff und/oder Kohlenmonoxid eingesetzt wird.

9. Verfahren nach Anspruch 1 bis 8, **dadurch gekennzeichnet, dass** die Reaktion bei Temperaturen von 20 bis 400°C durchgeführt wird.
